(19) **Europäisches Patentamt**

**European Patent Office**

**Office européen des brevets**

(11) **EP 1 652 550 A1**

(12) **EUROPEAN PATENT APPLICATION**
published in accordance with Art. 158(3) EPC

(43) Date of publication:
**03.05.2006 Bulletin 2006/18**

(51) Int Cl.:
**A61M 29/02** (1968.09)

(21) Application number: **04748113.0**

(22) Date of filing: **23.07.2004**

(86) International application number:
**PCT/JP2004/010906**

(87) International publication number:
**WO 2005/011796 (10.02.2005 Gazette 2005/06)**

(84) Designated Contracting States:
**AT BE BG CH CY CZ DE DK EE ES FI FR GB GR HU IE IT LI LU MC NL PL PT RO SE SI SK TR**

(30) Priority: **05.08.2003 JP 2003287165**
**05.08.2003 JP 2003287164**

(71) Applicant: **KANEKA CORPORATION**
**Osaka-shi, Osaka 530-8288 (JP)**

(72) Inventors:
• **NISHIDE, Takuji,**
**Osaka Plant,**
**KANEKA CORPORATION**
**Settsu-shi,**
**Osaka 5660072 (JP)**
• **NAKANO, Ryoji,**
**Osaka Plant,**
**KANEKA CORPORATION**
**Settsu-shi,**
**Osaka 5660072 (JP)**

• **YOSHIDA, Shinya,**
**Osaka Plant,**
**KANEKA CORPORATION**
**Settsu-shi,**
**Osaka 5660072 (JP)**
• **FUKAYA, Kohei,**
**Osaka Plant,**
**KANEKA CORPORATION**
**Settsu-shi,**
**Osaka 5660072 (JP)**
• **KAWATSU, Masaji,**
**Takasago Plant,**
**Miyamaecho**
**Takasago-shi,**
**Hyogo 6768688 (JP)**

(74) Representative: **Gille Hrabal Struck Neidlein Prop Roos**
**Brucknerstrasse 20**
**40593 Düsseldorf (DE)**

(54) **STENT TO BE PLACED IN VIVO**

(57)    As a treatment for angiostenosis, angioplasty (PTA or PTCA) of expanding a small-sized balloon in a vessel has been commonly conducted. However, this treatment easily causes repeated stenosis (restenosis) after the treatment. Placement of a stent in a vessel is also effective in decreasing restenosis, but this treatment may also cause restenosis. The present invention provides a stent containing a poly (lactide-co-glycolide) or both a poly (lactide-co-glycolide) and an immunosuppressive agent in at least a portion of a surface of the stent, and further containing a material nondegradable in vivo.

FIG. 2

**EP 1 652 550 A1**

**Description**

**Technical Field**

The present invention relates to a medical stent for in vivo placement for use in preventing or treating excessive vascular proliferation.

**Background Art**

At present, one of the serious health problems that confront us is angiostenosis due to arteriosclerosis. As a treatment method for angiostenosis, angioplasty (PTA or PTCA) of expanding a small-sized balloon in a vessel has been commonly conducted as a minimally invasive treatment. However, this treatment causes repeated stenosis (restenosis) with high probability. As a method for decreasing the rate of restenosis, atherectomy, laser therapy, radiation therapy, or the like has been attempted, and another method such as a technique of placing a stent has been recently commonly employed.

In order to treat various diseases caused by stenosis or occlusion of a blood vessel or another lumen in vivo, a stent is mainly used as a medical device to be placed in a stenosed or occluded site, for expanding the site to maintain its lumen size, and a such a stent is generally composed of a metal or a polymer. A stent is generally inserted into a vessel through a catheter and is expanded in contact with a disease portion of an arterial wall, for mechanically supporting the intravascular lumen. Although it has been shown that the frequency of occurrence of restenosis is significantly decreased by stent placement, restenosis still occurs with high probability under the present condition. For example, with respect to the cardiac coronary artery, it has been reported that even when stent placement is performed, restenosis occurs at a frequency of about 20 to 30%. The restenosis may be induced by biological vascular damage or vascular damage due to stent placement. It is thought that typical vascular angiostenosis or restenosis induced by vascular damage is due to proliferation of smooth muscle cells in intima. Namely, the proliferation of smooth muscle cells in intima is started in succession to vascular damage, and then the smooth muscle cells are transferred to an intima. Next, the smooth muscle cells in intima proliferate accompanied with deposition on the substrate, thereby causing intimal thickening. It is also thought that T cells, macrophages, and the like are transferred to the intima.

In order to decrease the occurrence of restenosis after stent placement, various means have been investigated.

Conventional stents have been made of a metal such as stainless steel or tantalum, but polymer stents having a shape memory property have been studied, as disclosed in Patent Document 1. A polymer stent having a shape memory property is certainly expandable in a stenosed portion. However, the polymer stent has a problem in which control of the expansion size is difficult, and the strength to hold a stenosed vessel is insufficient because the stent is entirely made of a resin, thereby causing difficulty in holding the vessel for a long time, a problem in which the stent is brittle against bending, and a problem in which the polymer used is decomposed and eluted over a long period of time.

Patent Document 2 proposes a stent composed of a biodegradable polymer. Patent Document 3 also proposes a stent composed of a biodegradable polymer, and particularly discloses a stent composed of polylactic acid (PLA), polyglycolic acid (PGA), or a poly (lactide-co-glycolide). Such a stent composed of a biodegradable polymer completely disappears within a predetermined period after burying in a living body, and thus the problem of decomposition and elution of a polymer over a long period of time is resolved. However, the problem of insufficient stent strength and the problem of brittleness against bending remain unresolved. Furthermore, degradation of a biodegradable polymer proceeds even in production and processing, and thus a stent entirely composed of a biodegradable polymer exhibits large variations in strength in actual use. Therefore, from the viewpoint of stent strength, the effective period from production to use must be shortened. Although polylactic acid (PLA), polyglycolic acid (PGA), a poly (lactide-co-glycolide), and the like have excellent biocompatibility, they are known to cause inflammation in the surrounding tissues during degradation. Therefore, when such a polymer is used as a stent material, it is important to minimize the amount of the polymer used. The above-described conventional technique has the problem of difficulty in suppressing the amount of the biodegradable polymer used, for maintaining the strength of a stent which is entirely made of the biodegradable polymer.

Accordingly, there has been proposed an attempt to decrease the occurrence rate of restenosis by coating a stent with a drug for limiting obstruction (for example, Patent Document 4). As the drug for limiting obstruction, various drugs, such as an anticoagulant, an antiplatelet, an antibacterial drug, an antitumor drug, an antimicrobial drug, an anti-inflammatory agent, an antimetabolic drug, an immunosuppressive agent, and the like have been researched. With respect to the immunosuppressive agent, there have been proposed stents coated with cyclosporine, tacrolimus (FK-506), sirolimus (rapamycin), mycophenolate mofetil, and analogues thereof (everolimus, ABT-578, CCI-779, AP23573, etc.), for decreasing restenosis. Specific examples of such stents include a stent coated with sirolimus (rapamycin) known as an immunosuppressive agent as disclosed in Patent Document 5, and a stent coated with taxol (paclitaxel) serving as an antitumor agent as disclosed in Patent Document 6. Furthermore, for example, Patent Documents 7 and 8 disclose stents coated with tacrolimus (FK-506).

Tacrolimus (FK-506) is a compound of CAS No. 104987-11-3 and is disclosed in, for example, Patent Document 9. Tacrolimus (FK-506) possibly forms a complex with an intracellular FK506 binding protein (FKBP) to inhibit the production of cytokines such as IL-2, INE-γ, and the like, which mainly serve as a differentiation/proliferation factor, from T cells. It is well known that tacrolimus (FK-506) can be used as a preventive or curative agent for rejection in organ transplantation and for autoimmune disease. It is also confirmed that tacrolimus (FK-506) has an antiproliferative action on human vascular cells (Non-patent Document 1).

As a method for carrying a drug, Patent Document 4 discloses that a drug is carried using a polymer, and also discloses use of a biodegradable polymer. Patent Document 10 also discloses use of a biodegradable polymer and examples of the polymer, such as polylactic acid.

However, even in use of the above-described drug-coated stent, the frequency of occurrence of stenosis is still high under the present condition. Therefore, it is desired to decrease the occurrence rate of stenosis.

[Patent Document 1] Japanese Unexamined Patent Application Publication No. 3-21262
[Patent Document 2] Japanese Unexamined Patent Application Publication No. 5-103830
[Patent Document 3] Japanese Unexamined Patent Application Publication No. 9-308693
[Patent Document 4] PCT Japanese Translation Patent Publication No. 5-502179
[Patent Document 5] Japanese Unexamined Patent Application Publication No. 6-009390
[Patent Document 6] PCT Japanese Translation Patent Publication No. 9-503488
[Patent Document 7] Publication No. WO02/065947
[Patent Document 8] Publication No. EP1254674
[Patent Document 9] Japanese Unexamined Patent Application Publication No. 61-148181
[Patent Document 10] PCT Japanese Translation Patent Publication No. 5-509008
[Non-patent Document 1] Paul J. Mohacsi MD, et al., The Journal of Heart and Lung Transplantation, May 1997, Vol. 16, No. 5, 484-491

## Disclosure of the Invention

In consideration of the above-mentioned situation, an object of the present invention is to resolve the problems of conventional stents for in vivo placement and provide a stent for in vivo placement which is capable of decreasing the rate of occurrence of repeated stenosis (restenosis).

As a result of intensive research for resolving the above-mentioned problems, the inventors of the present invention invented a stent for in vivo placement, said stent comprising being formed in a substantially tubular shape and expandable in the outward radial direction of the substantially tubular shape containing a material nondegradable in vivo, a poly (lactide-co-glycolide) on at least a portion of the surface thereof. The poly (lactide-co-glycolide) is preferably on either the outer surface or the inner surface of the stent, and more preferably over substantially the entire surface including the outer surface, the inner surface, and the side surfaces thereof.

The weight-average molecular weight of the poly (lactide-co-glycolide) is preferably 5,000 to 130,000, and the molar ratios of lactic acid and glycolic acid which constitute the poly (lactide-co-glycolide) are preferably 50 mol% to 85 mol% and 15 mol% to 50 mol%, respectively.

The weight of the poly (lactide-co-glycolide) on the stent is preferably 3 μg/mm to 80 μg/mm and more preferably 7 μg/mm to 65 μg/mm per unit length in the axial direction of the stent.

As a result of intensive research, the inventors of the present invention also invented a stent for in vivo placement comprising being formed in a substantially tubular shape and expandable in the outward radial direction of the substantially tubular shape, containing a substrate nondegradable in vivo, and a poly (lactide-co-glycolide) and an immunosuppressive agent on at least a portion of the surface thereof. The poly (lactide-co-glycolide) and the immunosuppressive agent are preferably on either the outer surface or the inner surface of the stent, and more preferably over substantially the entire surface including the outer surface, the inner surface, and the side surfaces thereof.

The weight-average molecular weight of the poly (lactide-co-glycolide) is preferably 5,000 to 130,000, and the molar ratios of lactic acid and glycolic acid which constitute the poly (lactide-co-glycolide) are preferably 50 mol% to 85 mol% and 15 mol% to 50 mol%, respectively.

The immunosuppressive agent is preferably tacrolimus (FK-506), cyclosporine, sirolimus (rapamycin), azathioprine, mycophenolate mofetil, or an analogue thereof, and more preferably tacrolimus (FK-506).

The total weight of the poly (lactide-co-glycolide) and the immunosuppressive agent contained in the stent is preferably 3 μg/mm to 80 μg/mm and more preferably 7 μg/mm to 65 μg/mm per unit length in the axial direction of the stent.

The weight ratios of the poly (lactide-co-glycolide) and the immunosuppressive agent are preferably 30% by weight to 80% by weight and 20% by weight to 70% by weight, and more preferably 40% by weight to 70% by weight and 30% by weight to 60% by weight, respectively.

Also, an inner layer containing the poly (lactide-co-glycolide) and the immunosuppressive agent may be provided on a surface of the stent, and an outer layer containing only the poly (lactide-co-glycolide) may be provided on the outer surface of the inner layer.

The stent for in vivo placement according to the present invention is a stent containing a material nondegradable in vivo and further containing a poly (lactide-co-glycolide) or the poly (lactide-co-glycolide) and an immunosuppressive agent at least in a portion of a surface thereof. Therefore, the rate of occurrence of stenosis or restenosis, which occurs in a conventional stent for in vivo placement, can be decreased.

**Brief Description of the Drawings**

Fig. 1 is a developed view of a stent according to the present invention.
Fig. 2 is a schematic view of a stent according to the present invention.

**Best Mode for Carrying Out the Invention**

Embodiments of the present invention will be described below, but the present invention is not limited to these embodiments.

In a representative embodiment of the present invention, a stent for in vivo placement is formed in a substantially tubular shape and expandable in the outward radial direction of the substantially tubular shape, and contains a material nondegradable in vivo and further contains a poly (lactide-co-glycolide) in at least a portion of the surface thereof. For example, the stent can be formed by coating at least a portion of a surface of the stent with the poly (lactide-co-glycolide). Substantially the entire surface including the outer surface, the inner surface, and the side surfaces of the stent is preferably coated with the poly (lactide-co-glycolide). When the entire surface of the stent is coated with the poly (lactide-co-glycolide), platelets little adhere to the entire surface of the stent, and thus a stimulus to the surrounding tissues can be decreased. When only a portion of the surface of the stent is coated, the above-described function can be selectively expected only in the coated portion. In particular, when the outer surface of the stent is coated, the coating directly contacts the inner wall of a vessel, thereby possibly causing a direct action on the inner wall of the vessel. When the inner surface of the stent is coated, the coating can possibly act on a relatively wide region through the blood flowing through a vessel.

In another representative embodiment of the present invention, a stent for in vivo placement is formed in a substantially tubular shape and expandable in the outward radial direction of the substantially tubular shape, and contains a material nondegradable in vivo and also contains a poly (lactide-co-glycolide) and an immunosuppressive agent in at least a portion of a surface thereof. For example, the stent can be formed by coating at least a portion of the surface of the stent with the poly (lactide-co-glycolide) and the immunosuppressive agent. Substantially the entire surface including the outer surface, the inner surface, and the side surfaces of the stent is preferably coated with the poly (lactide-co-glycolide) and the immunosuppressive agent. When the entire surface of the stent is coated with the poly (lactide-co-glycolide) and the immunosuppressive agent, platelets little adhere to the entire surface of the stent, and thus a stimulus to the surrounding tissues can be decreased. When only a portion of the surface of the stent is coated with the poly (lactide-co-glycolide) and the immunosuppressive agent, the above-described function can be selectively expected only in the coated portion. In particular, when the outer surface of the stent is coated, the coating directly contacts the inner wall of a vessel, thereby possibly causing a direct action on the inner wall of the vessel. When the inner surface of the stent is coated, the coating can possibly act on a relatively wide region through the blood flowing through a vessel.

Preferred examples of the material nondegradable in vivo used in the present invention include metal materials, such as stainless steel, a Ni-Ti alloy, a Cu-Al-Mn alloy, tantalum, a Co-Cr alloy, indium, indium oxide, and niobium. (The material nondegradable in vivo used in the present invention is not strictly required to be nondegradable in vivo, and it is sufficient that the shape can be maintained over a relatively long period of time. Hereinafter, the term "substrate" may be used as a term indicating a portion made of a material nondegradable in vivo in the present invention.) The substrate of the stent can be formed by the same method as that commonly used by a person skilled in the art in which a cylindrical metal tube is cut into a stent design by laser cutting and then electrolytically polished. However, the forming method is not limited to this, and an etching method, a method including cutting a plate metal with a laser, rounding the plate, and then welding, a method of knitting a metal wire, or the like can be also used. In the present invention, the material nondegradable in vivo is not limited to metal materials, and other usable examples include polymer materials, such as polyolefins, polyolefin elastomers, polyamides, polyamide elastomers, polyurethanes, polyurethane elastomers, polyesters, polyester elastomers, polyimides, polyamide-imides, and polyether ether ketones; and inorganic materials, such as ceramics and hydroxyapatite. A method for forming the stent substrate using such a polymer material or inorganic material does not restrict the advantage of the present invention, and any desired processing method suitable for each material can be arbitrarily selected. Since the stent of the present invention contains the nondegradable material, the strength shortage of the stent can be prevented, and variations in strength of the stent in actual use can be decreased. The nondegradable material is more preferably disposed so as to form the skeleton of the stent.

In the representative embodiment of the present invention in which only the poly (lactide-co-glycolide) is contained, the weight-average molecular weight of the poly (lactide-co-glycolide) is preferably 5,000 to 130,000. The molar ratios of lactic acid and glycolic acid which constitute the poly (lactide-co-glycolide) are preferably 50 mol% to 85 mol% and 15 mol% to 50 mol%, respectively. By controlling the weight-average molecular weight and the molar ratios of lactic acid and glycolic acid in the respective ranges described above, the biodegradation rate of the poly (lactide-co-glycolide) can be controlled, thereby realizing a low rate of restenosis.

In use of the poly (lactide-co-glycolide) having a weight-average molecular weight of 5,000 to 130,000 and the lactic acid and glycolic acid molar ratios of 50 mol% to 85 mol% and 15 mol% to 50 mol%, respectively, restenosis within and around the stent can be suppressed by a balance between tissue stimulation, degradation rate, and the like. This is remarkable in comparison to a stent not containing a poly (lactide-co-glycolide). Also, by controlling the weight-average molecular weight and the molar ratios of lactic acid and glycolic acid in the respective ranges described above, the biodegradation rate of the poly (lactide-co-glycolide) can be controlled, thereby realizing a low rate of restenosis.

The weight of the poly (lactide-co-glycolide) contained in the stent is preferably 3 $\mu$g/mm to 80 $\mu$g/mm per unit length in the axial direction of the stent, and more preferably 7 $\mu$g/mm to 65 $\mu$g/mm per unit length in the axial direction of the stent. When the weight of the poly (lactide-co-glycolide) contained in the stent is excessively small, the effect thereof is low, and the rate of restenosis is substantially the same as in a case not using the poly (lactide-co-glycolide). Conversely, when the weight is excessively large, like in a stent entirely composed of only the poly (lactide-co-glycolide), inflammatory reaction accompanying degradation of the poly (lactide-co-glycolide) becomes excessive, thereby relatively increasing the rate of restenosis. When the weight of the poly (lactide-co-glycolide) contained in the stent is 3 $\mu$g/mm to 80 $\mu$g/mm per unit length in the axial direction of the stent, as described above, the rate of restenosis is decreased, as compared with a stent not containing the poly (lactide-co-glycolide). When the weight of the poly (lactide-co-glycolide) contained in the stent is 7 $\mu$g/mm to 65 $\mu$g/mm per unit length in the axial direction of the stent, the effect becomes more significant.

In the other representative embodiment of the present invention which includes the poly (lactide-co-glycolide) and the immunosuppressive agent, the weight-average molecular weight of the poly (lactide-co-glycolide) is preferably 5,000 to 130,000. The molar ratios of lactic acid and glycolic acid which constitute the poly (lactide-co-glycolide) are preferably 50 mol% to 85 mol% and 15 mol% to 50 mol%, respectively. By controlling the weight-average molecular weight and the molar ratios of lactic acid and glycolic acid in the respective ranges described above, the biodegradation rate of the poly (lactide-co-glycolide) can be controlled, and the immunosuppressive agent contained in the stent can be efficiently transferred to a target portion to be treated. As a result, a very low rate of restenosis can be realized.

In use of the substrate including the poly (lactide-co-glycolide) having a weight-average molecular weight of 5,000 to 130,000 and the lactic acid and glycolic acid molar ratios of 50 mol% to 85 mol% and 15 mol% to 50 mol%, respectively, restenosis within and around the stent can be suppressed by a balance between tissue stimulation, degradation rate, and the like. This is remarkable in comparison to a stent not containing a poly (lactide-co-glycolide). Also, by controlling the weight-average molecular weight and the molar ratios of lactic acid and glycolic acid in the respective ranges described above, the biodegradation rate of the poly (lactide-co-glycolide) can be controlled, and the immunosuppressive agent contained in the stent can be efficiently transferred to a target portion to be treated, thereby realizing a very low rate of restenosis.

As the immunosuppressive agent, tacrolimus (FK-506), cyclosporine, sirolimus (rapamycin), azathioprine, mycophenolate mofetil, or an analogue thereof (everolimus, ABT-578, CCI-779, AP23573, or the like) can be used, but tacrolimus (FK-506) is particularly preferably used.

The total weight of the poly (lactide-co-glycolide) and the immunosuppressive agent contained in the stent is preferably 3 $\mu$g/mm to 80 $\mu$g/mm per unit length in the axial direction of the stent and more preferably 7 $\mu$g/mm to 65 $\mu$g/mm per unit length in the axial direction of the stent. When the total amount of the poly (lactide-co-glycolide) and the immunosuppressive agent contained in the stent is excessively small, the effect is low, and the rate of restenosis is substantially the same as that of a stent not containing the poly (lactide-co-glycolide) and the immunosuppressive agent. Conversely, when the amount is excessively large, a high volume of the immunosuppressive agent can be transferred to a portion to be treated, but like in a stent entirely made of the poly (lactide-co-glycolide), inflammatory reaction accompanying degradation of the poly (lactide-co-glycolide) becomes excessive, thereby relatively increasing the rate of restenosis. When the total weight of the poly (lactide-co-glycolide) and the immunosuppressive agent contained in the stent is 3 $\mu$g/mm to 80 $\mu$g/mm per unit length in the axial direction of the stent, as described above, the rate of stenosis is decreased, as compared with a stent not containing the poly (lactide-co-glycolide) and the immunosuppressive agent. When the weight is 7 $\mu$g/mm to 65 $\mu$g/mm, the effect becomes more significant.

The weight ratios of the poly (lactide-co-glycolide) and the immunosuppressive agent are preferably in ranges of 30% by weight to 80% by weight and 20% by weight to 70% by weight, and more preferably in rages of 40% by weight to 70% by weight and 30% by weight to 60% by weight, respectively. Since the ratios of the poly (lactide-co-glycolide) and the immunosuppressive agent influence the release rate of the immunosuppressive agent and the immunosuppressive agent-carrying capacity, the ratios greatly influence the rate of stenosis in the stent. When the ratios of the poly (lactide-co-glycolide) and the immunosuppressive agent are less than 30% by weight and more than 70% by weight, respectively, the immunosuppressive agent-carrying capacity of the stent is relatively increased, but the immunosuppressive agent is released at a high rate and becomes difficult to release over a long time, thereby failing to sufficiently suppress restenosis. When the ratios of the poly (lactide-co-glycolide) and the immunosuppressive agent are more than 80% by weight and less than 20% by weight, respectively, the immunosuppressive agent can be released over a long time, but the immunosuppressive agent-carrying capacity of the stent is relatively decreased. When a sufficient amount of the immunosuppressive agent is carried for suppressing restenosis, the amount of the poly (lactide-co-glycolide) is significantly increased, and thus inflammatory reaction accompanying degradation of the poly (lactide-co-glycolide) becomes excessive, thereby possibly increasing the rate of restenosis.

Furthermore, when the stent has an inner layer provided on a surface thereof and including the poly (lactide-co-glycolide) containing the immunosuppressive agent, and an outer layer provided on the outer surface of the inner layer and including only the poly (lactide-co-glycolide), the sustained release of the immunosuppressive agent can be further improved. In this case, the sustained release of the immunosuppressive agent can be controlled by adjusting the thickness of the outer layer and the molar ratios of lactic acid and glycolic acid which constitute the poly (lactide-co-glycolide).

A method usable for applying the poly (lactide-co-glycolide) to the substrate of the stent may be any one of various methods, such as a method including dissolving the poly (lactide-co-glycolide) in a solvent, attaching the resultant solution to the substrate, and then removing the solvent, a method of bonding a separately prepared film of the poly (lactide-co-glycolide) to the substrate, and the like.

As the method of attaching the solution of the poly (lactide-co-glycolide) to the substrate, a method of dipping the substrate in the solution, a method of spraying the solution on the substrate, or the like can be used. As the solvent used for preparing the solution, any solvent can be selected as long as the poly (lactide-co-glycolide) is soluble in the solvent. In order to control volatility and the like, a mixture of two or more solvents may be used. Also, the concentration of the poly (lactide-co-glycolide) is not particularly limited, and any concentration may be used in consideration of the surface quality and the like after application. Furthermore, in order to control the surface quality after application, the residual solution may be removed during and/or after the attachment of the solution of the poly (lactide-co-glycolide) to the substrate. Examples of removing means include vibration, rotation, pressure reduction, and the like. These means may be used in combination of two or more.

A method usable for applying the poly (lactide-co-glycolide) and the immunosuppressive agent to the substrate of the stent may be any one of various methods, such as a method including dissolving the poly (lactide-co-glycolide) and the immunosuppressive agent in a solvent, attaching the resultant solution to the substrate, and then removing the solvent; a method including dissolving only the immunosuppressive agent in a solvent, attaching the resultant solution to the substrate, removing the solvent to apply the immunosuppressive agent, attaching a solution of the poly (lactide-co-glycolide), and then removing the solvent; a method of bonding a separately prepared film of the poly (lactide-co-glycolide) containing the immunosuppressive agent to the substrate; a method including applying only the immunosuppressive agent to the substrate and then bonding a film of the poly (lactide-co-glycolide); and the like.

As the method of attaching the solution of the poly (lactide-co-glycolide) and/or the immunosuppressive agent to the substrate, a method of dipping the substrate in the solution, a method of spraying the solution on the substrate, or the like can be used. When the poly (lactide-co-glycolide) and the immunosuppressive agent are simultaneously attached in a solution state to the substrate, any solvent can be selected as the solvent used for preparing the solution as long as the poly (lactide-co-glycolide) and the immunosuppressive agent are soluble in the solvent. When the poly (lactide-co-glycolide) and the immunosuppressive agent are separately attached in a solution state to the substrate, any solvent can be selected as the solvent used for preparing the solution as long as either of the poly (lactide-co-glycolide) and the immunosuppressive agent is soluble in the solvent. In any case, in order to control volatility and the like, a mixture of two or more solvents may be used. Also, the concentration of the poly (lactide-co-glycolide) and/or the immunosuppressive agent is not particularly limited, and any concentration may be used in consideration of the surface quality after application, the release behavior of the immunosuppressive agent, and the like. Furthermore, in order to control the surface quality after application, the residual solution may be removed during and/or after the attachment of the solution of the poly (lactide-co-glycolide) and/or the immunosuppressive agent to the substrate. Examples of removing means include vibration, rotation, pressure reduction, and the like. These means may be used in combination of two or more.

**[Examples]**

**(Example 1)**

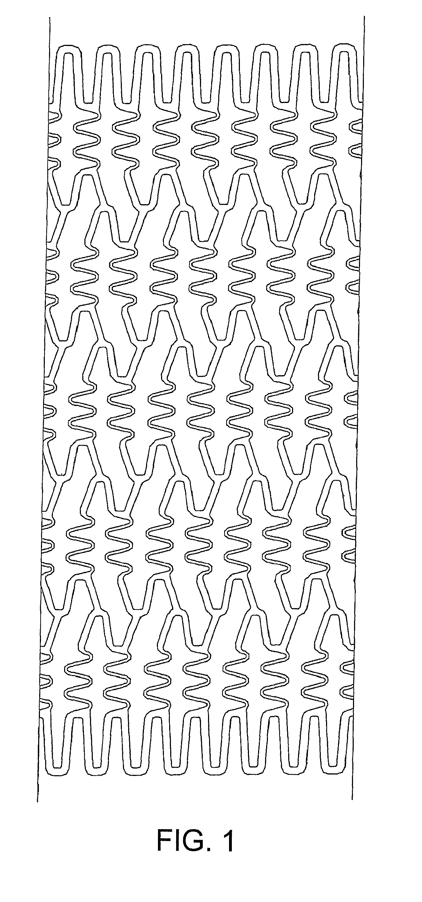

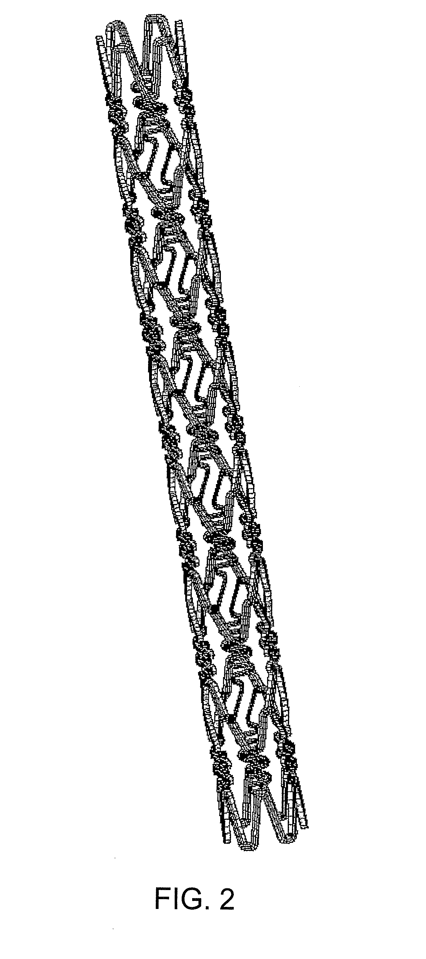

A substrate of a stent was formed by the same method as that commonly used by a person skilled in the art in which a stainless steel (SUS316L) cylindrical tube having an inner diameter of 1.50 mm and an outer diameter of 1.80 mm was cut into a stent design by laser cutting, and then electrolytically polished. Fig. 1 is a developed view of the stent used, and Fig. 2 is a schematic view. The stent had a length of 13 mm, a thickness of 120 $\mu$m, and a nominal diameter after expansion of 3.5 mm. The stent was a so-called balloon expandable type in which the stent is expanded and placed using a balloon catheter having a balloon provided near the tip thereof. The balloon expandable type stent is set in a contracted state at the balloon of the balloon catheter, delivered to a target portion, and then expanded and placed by expansion of the balloon.

A poly (lactide-co-glycolide) (SIGMA Corp., lactic acid/glycolic acid = 85/15, weight-average molecular weight 90,000 to 126,000) was dissolved in chloroform (Wako Pure Chemical Industries, Ltd.) to prepare a 0.5 wt% solution. A stainless steel wire of 100 $\mu$m in diameter was fixed at one of the ends of the stent, and the other end of the stent was connected to a stirrer to hold the stent vertically in the length direction. The prepared solution was attached to the stent by spraying the solution on the stent using a spray gun having a nozzle diameter of 0.3 mm while the stirrer was rotated at 100 rpm. The distance between the nozzle of the spray gun and the stent was 75 mm, and the air pressure for spraying was 0.15 MPa. The sprayed solution was dried under vacuum at room temperature for 1 hour. The spray time was controlled so that the weight of the poly (lactide-co-glycolide) per unit length in the axial direction of the substrate was 3 $\mu$g/mm (39 $\mu$g per stent) to prepare a stent.

**(Example 2)**

A stent was prepared by the same method as in Example 1 except that the spray time was controlled so that the weight of the poly (lactide-co-glycolide) per unit length in the axial direction of the substrate was 7 $\mu$g/mm (91 $\mu$g per stent).

**(Example 3)**

A stent was prepared by the same method as in Example 1 except that the spray time was controlled so that the weight of the poly (lactide-co-glycolide) per unit length in the axial direction of the substrate was 65 $\mu$g/mm (845 $\mu$g per stent).

**(Example 4)**

A stent was prepared by the same method as in Example 1 except that the spray time was controlled so that the weight of the poly (lactide-co-glycolide) per unit length in the axial direction of the substrate was 80 $\mu$g/mm (1,040 $\mu$g per stent).

**(Example 5)**

A stent was prepared by the same method as in Example 1 except that the spray time was controlled so that the weight of the poly (lactide-co-glycolide) per unit length in the axial direction of the substrate was 3.5 $\mu$g/mm (45.5 $\mu$g per stent).

**(Example 6)**

A stent was prepared by the same method as in Example 1 except that the spray time was controlled so that the weight of the poly (lactide-co-glycolide) per unit length in the axial direction of the substrate was 10 $\mu$g/mm (130 $\mu$g per stent).

**(Example 7)**

A stent was prepared by the same method as in Example 1 except that the spray time was controlled so that the weight of the poly (lactide-co-glycolide) per unit length in the axial direction of the substrate was 32.5 $\mu$g/mm (423 $\mu$g per stent).

**(Example 8)**

A stent was prepared by the same method as in Example 1 except that the spray time was controlled so that the weight of the poly (lactide-co-glycolide) per unit length in the axial direction of the substrate was 40 $\mu$g/mm (520 $\mu$g per stent).

**(Example 9)**

A stent was prepared by the same method as in Example 1 except that a different poly (lactide-co-glycolide) (Wako Pure Chemical Industries, Ltd., lactic acid/glycolic acid = 50/50, weight-average molecular weight 5,000) was used, and the spray time was controlled so that the weight of the poly (lactide-co-glycolide) per unit length in the axial direction of the substrate was 7 $\mu$g/mm (91 $\mu$g per stent).

**(Example 10)**

A stent was prepared by the same method as in Example 9 except that a different poly (lactide-co-glycolide) (Polysciences Inc., lactic acid/glycolic acid = 50/50, weight-average molecular weight 12,000 to 16,500) was used.

**(Example 11)**

A stent was prepared by the same method as in Example 9 except that a different poly (lactide-co-glycolide) (Polysciences Inc., lactic acid/glycolic acid = 50/50, weight-average molecular weight 16,500 to 22,000) was used.

**(Example 12)**

A stent was prepared by the same method as in Example 9 except that a different poly (lactide-co-glycolide) (SIGMA Corp., lactic acid/glycolic acid = 50/50, weight-average molecular weight 40,000 to 75,000) was used.

**(Example 13)**

A stent was prepared by the same method as in Example 9 except that a different poly (lactide-co-glycolide) (SIGMA Corp., lactic acid/glycolic acid = 75/25, weight-average molecular weight 90,000 to 126,000) was used.

**(Example 14)**

A stent was prepared by the same method as in Example 9 except that a different poly (lactide-co-glycolide) (SIGMA Corp., lactic acid/glycolic acid = 65/35, weight-average molecular weight 40,000 to 75,000) was used.

**(Example 15)**

A substrate of a stent was formed by the same method as that commonly used by a person skilled in the art in which a stainless steel (SUS316L) cylindrical tube having an inner diameter of 1.50 mm and an outer diameter of 1.80 mm was cut into a stent design by laser cutting, and then electrolytically polished. Fig. 1 is a developed view of the stent used, and Fig. 2 is a schematic view. The stent had a length of 13 mm, a thickness of 120 $\mu$m, and a nominal diameter after expansion of 3.5 mm. The stent was a so-called balloon expandable type in which the stent is expanded and placed using a balloon catheter having a balloon provided near the tip thereof. The balloon expandable type stent is set in a contracted state at the balloon of the balloon catheter, delivered to a target portion, and then expanded and placed by expansion of the balloon.

A poly (lactide-co-glycolide) (SIGMA Corp., lactic acid/glycolic acid = 85/15, weight-average molecular weight 90,000 to 126,000) and an immunosuppressive agent (tacrolimus, Fujisawa Pharmaceutical Co., Ltd.) were dissolved in chloroform to prepare a solution containing 0.5 wt% of each component. A stainless steel wire of 100 $\mu$m in diameter was fixed at one of the ends of the stent, and the other end of the stent was connected to a stirrer to hold the stent vertically in the length direction. The prepared solution was attached to the stent by spraying the solution on the stent using a spray gun having a nozzle diameter of 0.3 mm while the stirrer was rotated at 100 rpm. The distance between the nozzle of the spray gun and the stent was 75 mm, and the air pressure for spraying was 0.15 MPa. The sprayed solution was dried under vacuum at room temperature for 1 hour. The spray time was controlled so that the total weight of the poly (lactide-co-glycolide) and the immunosuppressive agent per unit length in the axial direction of the substrate was 3 $\mu$g/mm (poly (lactide-co-glycolide)/immunosuppressive agent = 50/50, 39 $\mu$g per stent) to prepare a stent.

**(Example 16)**

A stent was prepared by the same method as in Example 15 except that the spray time was controlled so that the total weight of the poly (lactide-co-glycolide) and the immunosuppressive agent per unit length in the axial direction of the substrate was 7 $\mu$g/mm (91 $\mu$g per stent).

**(Example 17)**

A stent was prepared by the same method as in Example 15 except that the spray time was controlled so that the total weight of the poly (lactide-co-glycolide) and the immunosuppressive agent per unit length in the axial direction of the substrate was 20 $\mu$g/mm (260 $\mu$g per stent).

**(Example 18)**

A stent was prepared by the same method as in Example 15 except that the spray time was controlled so that the total weight of the poly (lactide-co-glycolide) and the immunosuppressive agent per unit length in the axial direction of the substrate was 65 $\mu$g/mm (845 $\mu$g per stent).

**(Example 19)**

A stent was prepared by the same method as in Example 15 except that the spray time was controlled so that the total weight of the poly (lactide-co-glycolide) and the immunosuppressive agent per unit length in the axial direction of the substrate was 80 $\mu$g/mm (1,040 $\mu$g per stent).

**(Example 20)**

A stent was prepared by the same method as in Example 15 except that a different poly (lactide-co-glycolide) (Wako Pure Chemical Industries, Ltd., lactic acid/glycolic acid = 50/50, weight-average molecular weight 5,000) was used, and the spray time was controlled so that the total weight of the poly (lactide-co-glycolide) and the immunosuppressive agent per unit length in the axial direction of the substrate was 20 $\mu$g/mm (260 $\mu$g per stent).

**(Example 21)**

A stent was prepared by the same method as in Example 20 except that a different poly (lactide-co-glycolide) (Polysciences Inc., lactic acid/glycolic acid = 50/50, weight-average molecular weight 12,000 to 16,500) was used.

**(Example 22)**

A stent was prepared by the same method as in Example 20 except that a different poly (lactide-co-glycolide) (Polysciences Inc., lactic acid/glycolic acid = 50/50, weight-average molecular weight 16,500 to 22,000) was used.

**(Example 23)**

A stent was prepared by the same method as in Example 20 except that a different poly (lactide-co-glycolide) (SIGMA Corp., lactic acid/glycolic acid = 50/50, weight-average molecular weight 40,000 to 75,000) was used.

**(Example 24)**

A stent was prepared by the same method as in Example 20 except that a different poly (lactide-co-glycolide) (SIGMA Corp., lactic acid/glycolic acid = 65/35, weight-average molecular weight 40,000 to 75,000) was used.

**(Example 25)**

A stent was prepared by the same method as in Example 20 except that a different poly (lactide-co-glycolide) (SIGMA Corp., lactic acid/glycolic acid = 75/25, weight-average molecular weight 40,000 to 75,000) was used.

**(Example 26)**

A stent was prepared by the same method as in Example 25 except that the concentration of the poly (lactide-co-glycolide) was 0.5 wt%, the concentration of the immunosuppressive agent was 1.17 wt%, and the spray time was controlled so that the total weight of the poly (lactide-co-glycolide) and the immunosuppressive agent per unit length in the axial direction of the substrate was about 14 μg/mm (186 μg per stent, poly (lactide-co-glycolide)/immunosuppressive agent = 30/70).

**(Example 27)**

A stent was prepared by the same method as in Example 25 except that the concentration of the poly (lactide-co-glycolide) was 0.5 wt%, the concentration of the immunosuppressive agent was 0.75 wt%, and the spray time was controlled so that the total weight of the poly (lactide-co-glycolide) and the immunosuppressive agent per unit length in the axial direction of the substrate was about 17 μg/mm (217 μg per stent, poly (lactide-co-glycolide)/immunosuppressive agent = 40/60).

**(Example 28)**

A stent was prepared by the same method as in Example 25 except that the concentration of the poly (lactide-co-glycolide) was 0.5 wt%, the concentration of the immunosuppressive agent was 0.21 wt%, and the spray time was controlled so that the total weight of the poly (lactide-co-glycolide) and the immunosuppressive agent per unit length in the axial direction of the substrate was about 33 μg/mm (433 μg per stent, poly (lactide-co-glycolide)/immunosuppressive agent = 70/30).

**(Example 29)**

A stent was prepared by the same method as in Example 25 except that the concentration of the poly (lactide-co-glycolide) was 0.5 wt%, the concentration of the immunosuppressive agent was 0.125 wt%, and the spray time was controlled so that the total weight of the poly (lactide-co-glycolide) and the immunosuppressive agent per unit length in the axial direction of the substrate was about 50 μg/mm (650 μg per stent, poly (lactide-co-glycolide)/immunosuppressive agent = 80/20).

**(Example 30)**

A stent was prepared by the same method as in Example 17 except that sirolimus (SIGMA Corp.) was used as the immunosuppressive agent.

**(Example 31)**

A stent was prepared by the same method as in Example 17 except that cyclosporine (Ciba Geigy Co., Ltd.) was used as the immunosuppressive agent.

**(Example 32)**

A 0.5 wt% chloroform solution of a poly (lactide-co-glycolide) (SIGMA Corp., lactic acid/glycolic acid = 85/15, weight-average molecular weight 90,000 to 126,000) was sprayed on the stent prepared in Example 17 to provide a poly (lactide-co-glycolide) layer (weight per unit length in the axial direction of the substrate: 7 μg/mm) not containing the immunosuppressive agent on the outer surface of the stent of Example 17.

**(Example 33)**

A stent was prepared by the same method as in Example 1 except that the spray time was controlled so that the weight of the poly (lactide-co-glycolide) per unit length in the axial direction of the substrate was 1 μg/mm (13 μg per stent).

**(Example 34)**

A stent was prepared by the same method as in Example 1 except that the spray time was controlled so that the weight of the poly (lactide-co-glycolide) per unit length in the axial direction of the substrate was 100 $\mu$g/mm (1,300 $\mu$g per stent).

**(Example 35)**

A stent was prepared by the same method as in Example 1 except that the spray time was controlled so that the weight of the poly (lactide-co-glycolide) per unit length in the axial direction of the substrate was 1.5 $\mu$g/mm (19.5 $\mu$g per stent).

**(Comparative Example 1)**

A substrate not coated with a poly (lactide-co-glycolide) was prepared.

**(Comparative Example 2)**

A stent was prepared by the same method as in Example 5 except that polylactic acid (Polysciences Inc., weight-average molecular weight 1,600 to 2,400) was used in place of the poly (lactide-co-glycolide).

**(Comparative Example 3)**

A stent was prepared by the same method as in Example 5 except that polylactic acid (Polysciences Inc., weight-average molecular weight 325,000 to 460,000) was used in place of the poly (lactide-co-glycolide).

**(Comparative Example 4)**

A stent was prepared by the same method as in Example 17 except that polylactic acid (Polysciences Inc., weight-average molecular weight 1,600 to 2,400) was used in place of the poly (lactide-co-glycolide).

**(Comparative Example 5)**

A stent was prepared by the same method as in Example 17 except that polylactic acid (Polysciences Inc., weight-average molecular weight 325,000 to 460,000) was used in place of the poly (lactide-co-glycolide).

(Placement experiment using mini swines)

Experiment of stent placement in mini swines (Clawn, female, 8 to 12 months old) was carried out using each of the stents described above to evaluate the stents. A sheath (6Fr) was inserted into the right femoral artery of each mini swine under anesthesia, and the tip of a guiding catheter (6Fr) inserted from the sheath was engaged with the ostium of the left coronary artery. Each stent was delivered to the anterior descending branch of the left coronary artery and the circumflex branch thereof through the guiding catheter and then expanded and placed. After the guiding catheter and the sheath were removed, the right femoral artery was ligated to perform hemostasis. The portion where the stent was placed had a vessel diameter of about 2.80 mm, and the expansion diameter of the stent was 3.50 mm so that the ratio of stent diameter/vessel diameter in the portion of placement was about 1.25. When a portion with a vessel diameter of 2.80 mm could not be selected, the expansion pressure of the balloon for expanding and placing the stent was changed so as to control the ratio of stent diameter/vessel diameter to about 1.25. In the experiment, the inner diameter of the stent was defined as the stent expansion diameter. When it was decided that the stent was difficult to expand and place in the anterior descending branch of the left coronary artery or the circumflex branch thereof due to the vessel diameter and a problem with vessel running, placement of the stent in this portion was canceled, and the stent was additionally placed in the right coronary artery. The number of the stents placed per mini swine was not limited.

The mini swines were administered with aspirin and ticlopidine in doses of 330 mg/day and 250 mg/day, respectively, by mixing with feedstuff from a day before the placement experiment to autopsy. One month after the placement, the mini swines were euthanized, and the heart was extracted from each mini swine. The coronary artery in which the stent was placed was extracted from the heart and immersed and fixed in a 10% neutral buffered formalin solution. After resin embedding, a section was cut out from the central portion of each stent and stained by H. E. (hematoxylin-eosin) and E. V. G. (Elastica-van Gieson), followed by magnification observation. As evaluation items, the lumen area (LA) and area within the internal elastic lamina (IELA) of each stent section were measured. The vascular occlusion rate of each stent was calculated using the lumen area (LA) and area within the internal elastic lamina (IELA) according to the equation below. Three stents of each of Examples 1 to 32 and Comparative Examples 1 to 8 were used in the placement experiment. The evaluation results are shown in Tables 1 to 5.

$$\text{Vascular occlusion rate (\%) = (1 - (LA/IELA))} \times 100$$

(Evaluation results)

    Table 1

    Table 2

    Table 3

    Table 4

    Table 5

Table 1 indicates that the stents of Examples 1 to 8, 33, and 34 and Comparative Examples 2 and 3 each containing only the poly (lactide-co-glycolide) show low vascular occlusion rates and good results, as compared with the substrate of Comparative Example 1 not containing the poly (lactide-co-glycolide). In particular, in Examples 1 to 8, the vascular occlusion rates are 50% or less and satisfactory values. It is thus found that the weight of the poly (lactide-co-glycolide) per unit length in the axial direction of the substrate is preferably 3 $\mu$g/mm to 80 $\mu$g/mm and more preferably 7 $\mu$g/mm to 65 $\mu$g/mm.

Also, in Examples 2 and 9 to 14 and Comparative Examples 2 and 3 in each of which the stent for in vivo placement contains only the poly (lactide-co-glycolide), and the weight of the poly (lactide-co-glycolide) per unit length in the axial direction of the substrate is 7 $\mu$g/mm, the vascular occlusion rates are low and satisfactory values, as compared with the substrate of Comparative Example 1 not containing the poly (lactide-co-glycolide). In particular, in Examples 2 and 9 to 14, the vascular occlusion rates are 50% or less and satisfactory values. It is thus found that in the stent for in vivo placement containing only the poly (lactide-co-glycolide), the molar ratios of lactic acid and glycolic acid in the poly (lactide-co-glycolide) are preferably 50 mol% to 85 mol% and 15 mol% to 50 mol%, respectively. It is further found that the weight-average molecular weight of the poly (lactide-co-glycolide) is preferably 5,000 to 130,000.

Referring to Table 2, in Examples 15 to 19 in each of which the poly (lactide-co-glycolide) and the immunosuppressive agent are contained at the same weight, the vascular occlusion rates are significantly decreased, as compared with in Comparative Example 1 using only the substrate. This indicates the very excellent effect of the present invention. The results of Examples 15 to 19 show the effect superior to that of Examples 6 to 8 and 35 in each of which only the poly (lactide-co-glycolide) is contained. These results indicate that the total weight of the poly (lactide-co-glycolide) and the immunosuppressive agent per unit length in the axial direction of the substrate is preferably 3 $\mu$g/mm to 80 $\mu$g/mm. Furthermore, in Examples 16 to 18, the vascular occlusion rates are about 30% and more satisfactory values. This indicates that the total weight of the poly (lactide-co-glycolide) and the immunosuppressive agent per unit length in the axial direction of the substrate is more preferably 7 $\mu$g/mm to 65 $\mu$g/mm.

Referring to Table 3, in Examples 17 and 20 to 25 in each of which the poly (lactide-co-glycolide) and the immunosuppressive agent are contained, the vascular occlusion rates are significantly decreased, as compared with Comparative Examples 4 and 5. This indicates the excellent effect of the present invention. It is thus found that when the stent for in vivo placement contains the poly (lactide-co-glycolide) and the immunosuppressive agent, the weight-average molecular weight of the poly (lactide-co-glycolide) is preferably 5,000 to 130,000, and the molar ratios of lactic acid and glycolic acid constituting the poly (lactide-co-glycolide) are preferably 50 mol% to 85 mol% and 15 mol% to 50 mol%, respectively.

Referring to Table 4, in Examples 17 and 26 to 29 in each of which the poly (lactide-co-glycolide) and the immunosuppressive agent are contained, the vascular occlusion rates are significantly decreased, as compared with Example 7 in which only the poly (lactide-co-glycolide) is contained. This indicates the more excellent effect of the present invention. It is thus found that the weight ratios of the poly (lactide-co-glycolide) and the immunosuppressive agent are preferably in ranges of 30% by weight to 80% by weight and 20% by weight to 70% by weight, respectively. Furthermore, in Examples 27 and 28, the vascular occlusion rates are less than 20% and are extremely excellent values. It is thus found that the weight ratios of the poly (lactide-co-glycolide) and the immunosuppressive agent are more preferably in ranges of 40% by weight to 70% by weight and 30% by weight to 60% by weight, respectively.

Referring to Table 5, Examples 17 and 30 to 32 in each of which the poly (lactide-co-glycolide) and the immunosuppressive agent are contained show low vascular occlusion rates and a more excellent effect, as compared with Example 7 in which only the poly (lactide-co-glycolide) is contained. It is thus decided that the stenosis inhibiting effect of the poly (lactide-co-glycolide) and the immunosuppressive agent is sufficiently high. In particular, Examples 17 and 32 show a more excellent effect in comparison to Examples 30 and 31. It is thus found that tacrolimus is preferred as the immunosuppressive agent. In Example 32 in which the outer layer including only the poly (lactide-co-glycolide) is provided on the outer surface of the stent of Example 17, for controlling sustained release of tacrolimus, the effect is higher than that in Example 17. It is thus found that the substrate preferably has an inner layer provided on a surface thereof and including the poly (lactide-co-glycolide) containing the immunosuppressive agent, and an outer layer provided on the outer surface of the inner layer and including only the poly (lactide-co-glycolide).

**Industrial Applicability**

As described above, a stent for in vivo placement according to the present invention contains a material nondegradable in vivo and further includes a poly (lactide-co-glycolide) or both a poly (lactide-co-glycolide) and an immunosuppressive agent in at least a portion of a surface and preferably over the entire surface of the stent. As a result, the rate of occurrence of stenosis or restenosis which occurs in a conventional stent for in vivo placement can be decreased.

Table 1

| | Weight per length in axial direction of stent (μg/mm) | Molar ratio of lactic acid/glycolic acid | Weight-average molecular weight | Vascular occlusion rate one month after (%) |
|---|---|---|---|---|
| Example 1 | 3 | 85/15 | 90,000-126,000 | 48.1 |
| Example 2 | 7 | 85/15 | 90,000-126,000 | 42.2 |
| Example 3 | 65 | 85/15 | 90,000-126,000 | 40.7 |
| Example 4 | 80 | 85/15 | 90,000-126,000 | 45.6 |
| Example 5 | 3.5 | 85/15 | 90,000-126,000 | 45.7 |
| Example 6 | 10.0 | 85/15 | 90,000-126,000 | 44.3 |
| Example 7 | 32.5 | 85/15 | 90,000-126,000 | 41.5 |
| Example 8 | 40.0 | 85/15 | 90,000-126,000 | 46.2 |
| Example 9 | 7 | 50/50 | 5,000 | 49.8 |
| Example 10 | 7 | 50/50 | 12,000-16,500 | 44.4 |
| Example 11 | 7 | 50/50 | 16,500-22,000 | 41.7 |
| Example 12 | 7 | 50/50 | 40,000-75,000 | 44.6 |
| Example 13 | 7 | 75/25 | 90,000-126,000 | 38.9 |
| Example 14 | 7 | 65/35 | 40,000-75,000 | 49.3 |
| Example 33 | 1 | 85/15 | 90,000-126,000 | 63.1 |
| Example 34 | 100 | 85/15 | 90,000-126,000 | 58.4 |
| Comp. Example 1 | - | - | - | 66.8 |
| Comp. Example 1 | 7 | 100/0 | 1,600-2,400 | 57.2 |
| Comp. Example 1 | 7 | 100/0 | 325,000-460,000 | 59.0 |

EP 1 652 550 A1

Table 2

| | Weight of lactic acid-glycolic acid copolymer (μg/mm) | Weight ratio of lactic acid-glycolic acid copolymer/ immunosuppressive agent | | Weight of lactic acid-glycolic acid copolymer (μg) | Weight of immuno-suppressive agent (μg) | Total coating weight per unit length of stent (μg/mm) | Vascular occlusion rate (%) |
| | | Lactic acid-glycolic acid copolymer (wt%) | Immuno-suppressive agent (wt%) | | | | |
|---|---|---|---|---|---|---|---|
| Example 15 | 1.5 | 50 | 50 | 20 | 20 | 3 | 40.2 |
| Example 16 | 3.5 | 50 | 50 | 46 | 46 | 7 | 30.5 |
| Example 17 | 10.0 | 50 | 50 | 130 | 130 | 20 | 20.7 |
| Example 18 | 32.5 | 50 | 50 | 423 | 423 | 65 | 29.0 |
| Example 19 | 40.0 | 50 | 50 | 520 | 520 | 80 | 35.9 |
| Example 35 | 40.0 | 100 | 0 | 520 | 0 | 40.0 | 46.2 |
| Comp. Example 1 | - | - | - | - | - | - | 66.8 |

Immunosuppressive agent: tacrolimus (Examples 15 to 19), no (Comparative Example 1 and Example 35)

Lactic acid-glycolic acid copolymer:

composition ratio: lactic acid/glycolic acid =85/15, weight-average molecular weight: 90,000 to 126,000

EP 1 652 550 A1

Table 3

| | Lactic acid-glycolic acid copolymer composition | | | Vascular occlusion rate(%) |
|---|---|---|---|---|
| | Lactic acid (mol%) | Glycolic acid (mol%) | Weight-average molecular weight | |
| Example 17 | 85 | 15 | 90,000-126,000 | 20.7 |
| Example 20 | 50 | 50 | 5,000 | 38.9 |
| Example 21 | 50 | 50 | 12,000-16,500 | 36.6 |
| Example 22 | 50 | 50 | 16,500-22,000 | 34.2 |
| Example 23 | 50 | 50 | 40,000-75,000 | 25.2 |
| Example 24 | 65 | 35 | 40,000-75,000 | 23.1 |
| Example 25 | 75 | 25 | 90,000-126,000 | 28.7 |
| Comp. Example 4 | 100 | 0 | 1,600-2,400 | 63.2 |
| Comp. Example 5 | 100 | 0 | 325,000-460,000 | 59.1 |

Immunosuppressive agent: tacrolimus

Weight of lactic acid-glycolic acid copolymer per unit length of stent: 10 µg/mm

Weight of lactic acid-glycolic acid copolymer per stent: 130 µg

Weight of immunosuppressive agent per stent: 130 µg

Lactic acid-glycolic acid copolymer/immunosuppressive agent = 50/50

Total coating weight per unit length of stent: 20 µg/mm

EP 1 652 550 A1

Table 4

| | Weight ratio of lactic acid-glycolic acid copolymer/ immunosuppressive agent | | Weight of immuno- suppressive agent (μg) | Total coating weight per unit length of stent (μg/mm) | Vascular occlusion rate (%) |
|---|---|---|---|---|---|
| | Lactic acid- glycolic acid copolymer (wt%) | Immuno- suppressive agent (wt%) | | | |
| Example 17 | 50 | 50 | 130 | 20 | 20.7 |
| Example 26 | 30 | 70 | 56 | 14 | 25.5 |
| Example 27 | 40 | 60 | 87 | 17 | 19.7 |
| Example 28 | 70 | 30 | 303 | 33 | 18.5 |
| Example 29 | 80 | 20 | 520 | 50 | 30.1 |
| Example 7 | 100 | 0 | 0 | 32.5 | 41.5 |

Immunosuppressive agent: tacrolimus

Weight of lactic acid-glycolic acid copolymer per unit length of stent: 10 μg/mm

Lactic acid-glycolic acid copolymer composition: lactic acid/glycolic acid = 85/15

Weight-average molecular weight of lactic acid-glycolic acid copolymer: 90,000 to 126,000

Weight of lactic acid-glycolic acid copolymer per stent: 130 μg/mm

EP 1 652 550 A1

Table 5

| | Type of immuno-suppressive agent | Weight of immuno-suppressive agent per stent (µg) | Total coating weight per unit length of stent (µg/mm) | Vascular occlusion rate (%) |
|---|---|---|---|---|
| Example 17 | Tacrolimus | 130 | 20 | 30.7 |
| Example 30 | Sirolimus | 130 | 20 | 35.1 |
| Example 31 | Cyclosporine | 130 | 20 | 33.2 |
| Example 32 | Tacrolimus | 130 | 27 | 23.3 |
| Example 7 | - | 0 | 32.5 | 41.5 |

Example 32: A layer containing only the lactic acid-glycolic acid copolymer was applied to the outer surface of the stent of Example 17 (7 µg/mm).

Weight of lactic acid-glycolic acid copolymer per unit length of stent: 10 µg/mm

Weight of lactic acid-glycolic acid copolymer per stent: 130 µg

Lactic acid-glycolic acid copolymer composition: lactic acid/glycolic acid = 85/15

Weight-average molecular weight of lactic acid-glycolic acid copolymer: 90,000 to 126,000

Weight ratio of lactic acid-glycolic acid copolymer/immunosuppressive agent = 50/50

EP 1 652 550 A1

**Claims**

1. A stent for in vivo placement, said stent comprising being formed in a substantially tubular shape and expandable in the outward radial direction of the substantially tubular shape, containing a material nondegradable in vivo, and a poly (lactide-co-glycolide) on at least a portion of the surface thereof.

2. The stent according to claim 1, wherein the poly (lactide-co-glycolide) is on either the outer surface or the inner surface of the stent.

3. The stent according to claim 1, wherein the poly (lactide-co-glycolide) is over substantially the entire surface including the outer surface, the inner surface, and the side surfaces of the stent.

4. The stent according to any one of claims 1 to 3, wherein the weight-average molecular weight of the poly (lactide-co-glycolide) is 5,000 to 130,000.

5. The stent according to any one of claims 1 to 4, wherein the molar ratios of lactic acid and glycolic acid which constitute the poly (lactide-co-glycolide) are 50 mol% to 85 mol% and 15 mol% to 50 mol%, respectively.

6. The stent according to any one of claims 1 to 5, wherein the weight of the poly (lactide-co-glycolide) being on the stent is 3 $\mu$g/mm to 80 $\mu$g/mm per unit length in the axial direction of the stent.

7. The stent according to claim 6, wherein the weight of the poly (lactide-co-glycolide) being on the stent is 7 $\mu$g/mm to 65 $\mu$g/mm per unit length in the axial direction of the stent.

8. A stent for in vivo placement comprising being formed in a substantially tubular shape and expandable in the outward radial direction of the substantially tubular shape, containing a material nondegradable in vivo, and a poly (lactide-co-glycolide) and an immunosuppressive agent on at least a portion of the surface thereof.

9. The stent according to claim 8, wherein the poly (lactide-co-glycolide) and the immunosuppressive agent are on either the outer surface or the inner surface of the stent.

10. The stent according to claim 8, wherein the stent has the poly (lactide-co-glycolide) and the immunosuppressive agent are over substantially the entire surface including the outer surface, the inner surface, and the side surfaces of the stent.

11. The stent according to any one of claims 8 to 10, wherein the weight-average molecular weight of the poly (lactide-co-glycolide) is 5,000 to 130,000.

12. The stent according to any one of claims 8 to 11, wherein the molar ratios of lactic acid and glycolic acid which constitute the poly (lactide-co-glycolide) are 50 mol% to 85 mol% and 15 mol% to 50 mol%, respectively.

13. The stent according to any one of claims 8 to 12, wherein the immunosuppressive agent is tacrolimus (FK-506), cyclosporine, sirolimus (rapamycin), azathioprine, mycophenolate mofetil, or an analogue thereof.

14. The stent according to claim 13, wherein the immunosuppressive agent is tacrolimus (FK-506).

15. The stent according to any one of claims 8 to 14, wherein the total weight of the poly (lactide-co-glycolide) and the immunosuppressive agent contained in the stent is 3 $\mu$g/mm to 80 $\mu$g/mm per unit length in the axial direction of the stent.

16. The stent according to claim 15, wherein the total weight of the poly (lactide-co-glycolide) and the immunosuppressive agent being on the stent is 7 $\mu$g/mm to 65 $\mu$g/mm per unit length in the axial direction of the stent.

17. The stent according to any one of claims 8 to 16, wherein the weight ratios of the poly (lactide-co-glycolide) and the immunosuppressive agent are 30% by weight to 80% by weight and 20% by weight to 70% by weight, respectively.

18. The stent according to claim 17, wherein the weight ratios of the poly (lactide-co-glycolide) and the immunosuppressive agent are 40% by weight to 70% by weight and 30% by weight to 60% by weight, respectively.

**19.** The stent according to any one of claims 8 to 18, comprising an inner layer provided on a the surface of the stent, said inner layer containing the poly (lactide-co-glycolide) and the immunosuppressive agent, and an outer layer provided on the outer surface of the inner layer, said outer layer containing only the poly (lactide-co-glycolide).

FIG. 1

FIG. 2

<table>
<tr><td colspan="2"><strong>INTERNATIONAL SEARCH REPORT</strong></td><td>International application No.<br>PCT/JP2004/010906</td></tr>
</table>

**A. CLASSIFICATION OF SUBJECT MATTER**
    Int.Cl$^7$  A61M29/02

According to International Patent Classification (IPC) or to both national classification and IPC

**B. FIELDS SEARCHED**

Minimum documentation searched (classification system followed by classification symbols)
    Int.Cl$^7$  A61M29/02, A61F2/06

Documentation searched other than minimum documentation to the extent that such documents are included in the fields searched
    Jitsuyo Shinan Koho          1922–1996    Toroku Jitsuyo Shinan Koho    1994–2004
    Kokai Jitsuyo Shinan Koho    1971–2004    Jitsuyo Shinan Toroku Koho    1996–2004

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2002/056790 A2  (AVANTEC VASCULAR CORP.),<br>25 July, 2002 (25.07.02),<br>Full text; all drawings<br>& JP 2004-523275 A      & US 2002/0082679 A1 | 1-19 |
| X | JP 2002-95756 A  (Terumo Corp.),<br>02 April, 2002 (02.04.02),<br>Full text; all drawings<br>& US 2002/0035395 A1      & EP 1159934 A2 | 1-19 |

☐ Further documents are listed in the continuation of Box C.          ☐ See patent family annex.

| | | |
|---|---|---|
| \* | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search<br>  20 October, 2004 (20.10.04) | Date of mailing of the international search report<br>  09 November, 2004 (09.11.04) |
|---|---|
| Name and mailing address of the ISA/<br>    Japanese Patent Office | Authorized officer |
| Facsimile No. | Telephone No. |

Form PCT/ISA/210 (second sheet) (January 2004)